# NEW EUROPEAN PATENT SPECIFICATION

(11) **EP 0 710 645 B2**
(45) Date of publication and mention of the opposition decision: **17.07.2002**
(45) Mention of the grant of the patent: 18.08.1999
(21) Application number: 95116956.4
(22) Date of filing: 27.10.1995
(51) Int. Cl.: C07C 255/10

(54) **Perfluoro unsaturated nitrile compound and process for producing the same**
Perfluoro ungesättigten Nitrile und Verfahren zu ihrer Herstellung
Insaturés perfluoro nitriles et procédé pour leur préparation

(30) Priority: 04.11.1994 JP 29554894
(43) Date of publication of application: 08.05.1996
(73) Proprietor: NIPPON MEKTRON, LTD., Minato-ku Tokyo (JP); The Central Synthetic Rubbers Research Institute named for S.V. Lebedev, 198035 Saint Petersburg (RU)
(72) Inventor: Satoru, Saito, Kitaibaraki city, Ibaraki (JP); Riichi, Iwa, Kitaibaraki city, Ibaraki (JP); Haruyoshi, Tatsu, Hitachi city, Ibaraki (JP); Stefanovich, Rondarev Dmitrii, 198096, Saint Petersburg (RU); Vasilyevich, Sokolov Sergey, 198217 Saint Petersburg (RU); Vulfovich, Berenblit Vsevolod, 188663, Poselok Kuzmolovski (RU)
(74) Representative: Hansen, Bernd, Dr. Dipl.-Chem.

(56) References cited:
- EP-A- 0 174 614
- GB-A- 1 145 445
- US-A- 3 933 767
- US-A- 4 281 092
- CHEMICAL ABSTRACTS, vol. 113, no. 9, 1990 Columbus, Ohio, US; abstract no. 77752f, A.A. MACHULS ET AL. 'Radiochemical alkylation of adamantanes by perfluorovinyl ethers.' page 725; & KHIM. VYS. ENERG. , vol. 24, no. 2, 1990 pages 117-121,

## Description

### 1. FIELD OF THE INVENTION

The present invention relates to a method for producing fluorinated compounds carrying a carboxylic acid amide group. Said compounds are unsaturated perfluorovinyl ethers.

### 2. RELATED ART

Functionalized perfluorovinyl ethers of the general formula

CF₂=CF-O-(CF₂)ₙ-X

with -X being one of -CN, -COF, -COOH, -COOR₁, -COOM, and -CONR₂R₃ (M = Na, K, Cs; R₁ = C₁₋₁₀-alkyl; R₂ R₃ = H, R₁; n = 2 - 12) are disclosed in GB-A-1,145,445, together with a method for its production which is based on the pyrolysis of compounds of the formula

MOOC-(CF₂)ₙ-O-CF(CF₃)-COOM

under anhydrous conditions. The amide is prepared from the corresponding carboxylate with ammonia in anhydrous diethylether.

US-A-3,933,767 discloses cyanoperfluoro(vinyl ethers) of the formula

CF₂=CF-O-CF₂-(CF(CF₃)-O-CF₂)ₙ-CF(CN)-CF₃

with n = 0 - 4, and their use for the production of copolymers. The-preparation of these compounds is described, starting from hexafluoropropylene oxide and perfluoro(a-cyanopropionyl)fluoride, which is obtainable from carbonyl fluoride and perfluoroacrylonitrile according to the method disclosed in Ser. No. 262,591.

A curable copolymer comprising a repeating unit derived from the perfluoro(vinyl ether) of the formula

CF₂=CF-(O-CF₂-CF(CF₃))ₓ-O-(CF₂)ₙ-CN

with x = 1 - 2 and n = 1 - 4 is disclosed in US-A-4,281,092. The preparation of these compounds is taught by the combined teachings of JP-52-53,814 and US-4,138,426, from starting materials such as perfluoro-2-(2-fluorosulfonylethoxy)-propyl vinyl ether via the corresponding carboxylic acids and amides.

EP-A-0 174 614 describes polyfluoroalkenyl ethers of the formula

CFX=CX(CFX)ₙ-O-(CFZ-CF₂-O)ₘ-CFYZ.

with X = Cl, F; -Y = -COF, -COOM, -COOR, -CONR₂, or -CN (M = alkali metal; R = H, lower alkyl); Z = F, trifluoroalkyl; n = 2 - 6; n = 0 - 6, which do not suffer β-cleavage during polymerization. The amides are prepared by reacting the corresponding acid fluoride with an amine NHR₂. The solvent to be used in this reaction is not specified.

### SUMMARY OF THE INVENTION

It is an object of the present invention to provide a new method for producing perfluoro unsaturated carboxylic acid amide compounds of the formula

CF₂=CF-O-(CF₂)ₙ-O-CF(CF₃)-CONH₂ (II)

where n is an integer of 2, 3, 4 or 5.

### DETAILED DESCRIPTION OF THE INVENTION

The production of perfluoro vinyl ethers of formula (II) is disclosed in synthesis route (B).

### Synthesis route (A):

Perfluoro unsaturated carboxylic acid ester represented by the following general formula:

CF₂=CFO(CF₂)nOCF(CF₃)COOR

where R is an alkyl group having 1 to 10 carbon atoms and n is an integer of 2 to 5, is subjected to addition reaction of chlorine or bromine onto the vinyl group of the ester to obtain a halogenated perfluoro saturated carboxylic acid ester represented by the following general formula:

CF₂XCFXO(CF₂)nOCF(CF₃)COOR

where R and n have the same meanings as defined above and X is a chlorine atom or a bromine atom. Then, the thus obtained halogenated perfluoro saturated carboxylic acid ester is reacted with ammonia to convert the ester group to an acid amide group to obtain an acid amide represented by the following general formula:

CF₂XCFXO (CF₂)nOCF(CF₃)CONH₂

where X and n have the same meanings as defined above. Then, the acid amide is dehalogenated to form a vinyl group again and a unsaturated acid amide represented by the following general formula is obtained:

CF₂=CFO(CF₂)nOCF(CF₃)CONH₂

where n has the same meaning as defined above. Then, the resulting unsaturated acid amide is dehydrated to obtain a desired product.

The starting material for the synthesis route (A), i.e. perfluoro unsaturated carboxylic acid ester can be obtained by reacting a dicarboxylic acid fluoride represented by the following general formula:

FOC(CF₂)ₙ₋₂COF

where n is an integer of 2, 3, 4 or 5, with hexafluoropropene oxide to obtain a dicarboxylic acid difluoride represented by the following general formula [Angew. Chem. Int. Ed. 24, 161 ∼ 179 (1985)]:

FOCCF(CF₃)O(CF₂)nOCF(CF₃)COF

where n has the same meaning as defined above, and then by reacting only one acid fluoride group of the resulting dicarboxylic acid difluoride with alcohol to monoesterify the dicarboxylic acid difluoride to obtain a compound represented by the following general formula:

FOCCF(CF₃)O(CF₂)nOCF(CF₃)COOR

where n has the same meaning as defined above and R is an alkyl group having 1 to 10 carbon atoms, and then by subjecting the other remaining carboxylic acid fluoride group to a FCOF- releasing reaction.

In the addition reaction of halogen to the thus obtained perfluoro unsaturated carboxylic acid ester, chlorine or bromine is used as a halogen, where bromine is preferable from the viewpoint of easy handling and easy reaction controllability.

A fluorine-containing solvent such as 1,1,2-trichloro-1,2, 2-trifluoroethane (F-113), perfluoro (2-butyltetrahydrofuran), perfluorohexane, etc. is used. To activate the addition reaction, the reaction is also carried out under irradiation of light such as sun beams, ultraviolet lamp light, etc. in the presence of a fluorine-containing solvent.

Reaction of halogenated perfluoro saturated carboxylic acid ester with ammonia is carried out in the presence of such a solvent as used in the addition reaction of halogen. The reaction can be carried out under the atmospheric pressure or under pressure.

### Synthesis route (B):

The starting material, perfluoro unsaturated carboxylic acid ester, is directly reacted with ammonia.

The reaction of perfluoro unsaturated carboxylic acid ester with ammonia can be carried out in the same manner as in the reaction with ammonia in the above-mentioned Synthesis route (A). Since the perfluorovinyl group (CF₂=CF-) is not protected in that case, there is a possibility of side reaction to form an NC-CHF- group. Thus it is necessary to control reaction conditions, particularly to keep the amount of ammonia equimolar to the raw material ester.

### BRIEF DESCRIPTION OF THE DRAWINGS

Fig. 1 is a diagram showing infrared absorption spectra of methyl perfluoro (2-methyl-3,7-dioxa-8,9-dibromo-8-nonanoate).

Fig. 2 is a diagram showing infrared absorption spectra of methyl perfluoro (2-methyl-3,8-dioxa-9,10-dibromo-9-decanoate).

Fig. 3 is a diagram showing infrared absorption spectra of perfluoro (2-methyl-3, 8-dioxa-9, 10-dibromo-9-decanamide).

### PREFERRED EMBODIMENTS OF THE INVENTION

The present invention will be explained in detail below, referring to Examples.

### Reference Example 1 [Synthesis route (A)]

(1) 422g of methyl perfluoro(2-methyl-3,7-dioxa-8-nonanoate) having the following chemical formula:

   CF₂=CFO(CF₂)₃OCF(CF₃)COOCH₃

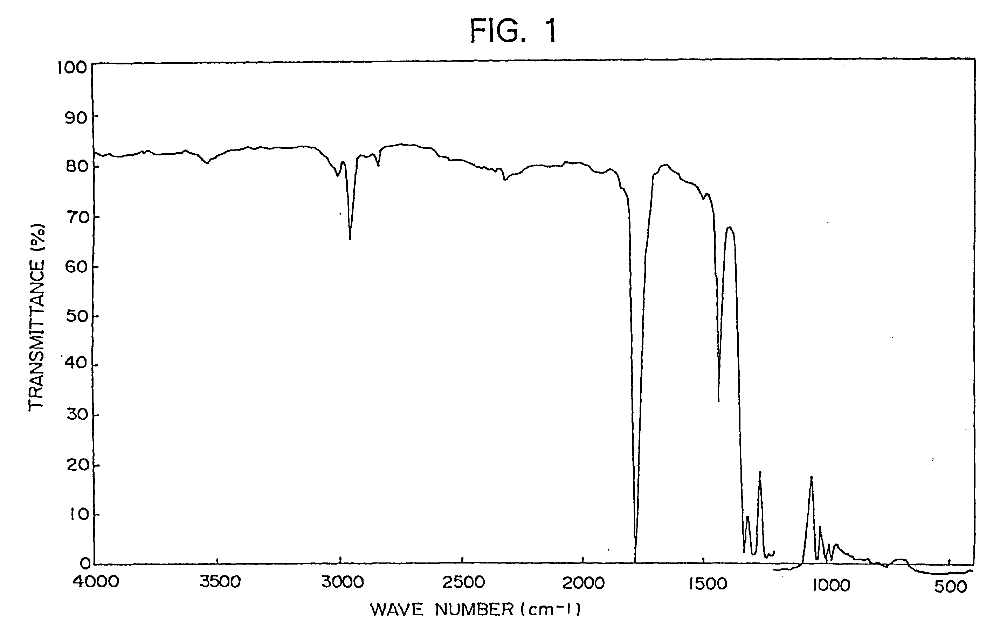

   and 300g of F-113 were charged into a three-necked glass flask having a net capacity of 1 liter, provided with a stirrer, a dropping funnel and a cooling pipe, and 160g of bromine was dropwise added thereto from the dropping funnel over one hour, while keeping the flask inside at 40°C. After the dropwise addition, the resulting reaction mixture was washed successively with an aqueous 5% sodium hydrogen sulfite solution and water. The organic layer was dried over magnesium sulfated, filtered and distilled, whereby 582g of methyl perfluoro(2-methyl-3,7-dioxa-8,9-dibromo-8-nonanoate) was obtained (yield: 100%).
   Boiling point: 87 ∼ 88°c (5 Torr),
   d₄²⁰: 1.8801, n_{D}²⁰: 1.3510
   Infrared absorption spectra: see Fig. 1
   ¹⁹F-NMR (internal standard: CFCl₃): 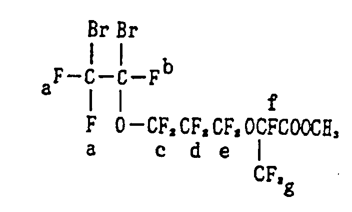
   a = -60.7ppm
   b =-68.5
   c =-83.5
   d =-126.7
   e = -76.1, -79.8
   f=-128.9
   g =-80.1
(2) 582g of the thus obtained methyl-perfluoro(2-methyl-3,7-dioxa-8,9-dibromo-8-nonancate) and 400g of F-113 were charged into a three-necked glass flask having a net capacity of 1 liter, provided with a stirrer, a gas feed tube and a dry ice cooling pipe, and 20g of ammonia gas was slowly introduced into the flask with stirring, while keeping the flask inside at a lower temperature than 30°C. After heat release was finished in the flask, stirring was further continued for 8 hours. Then, the solvent, ammonia and formed methanol were removed from the flask by distillation in reduced pressure, and then the residues were further distilled in reduced pressure, whereby 540g of perfluoro (2-methyl-3,7-dioxa-8,9-dibromo-8-nonanamide) having a melting point of 70 ∼ 75°C was obtained as white crystals (yield: 95%).

### Example 2 [Synthesis route (B)]

118g of methyl perfluoro(2-methyl-3,8-dioxa-9-decenate) having the following chemical formula:

CF₂=CFO(CF₂)₄OCF(CF₃)COOCH₃

and 200g of perfluoro(2-butyltetrahydrofuran) were charged into a three-necked glass flask having a net capacity of 500ml, provided with a stirrer, a gas feed tube and a dry ice cooling pipe, and 5g of ammonia gas was slowly introduced into the flask with stirring, while keeping the flask inside at a lower temperature than 0°C. After the ammonia addition, stirring was further continued for 3 hours, and the solvent, ammonia and formed methanol were removed therefrom by distillation under reduced pressure, whereby 80g of crude perfluoro(2-methyl-3,8-dioxa-9-decenamide) was obtained as white crystals. It was found from infrared absorption spectra (KBr) that the white crystals were a desired product by the presence of 1711cm⁻¹ (-CONH₂), 1841cm⁻¹ (C=C).

### Reference Example 3 [Synthesis route (A)]

(1) 33g of zinc powder and 200ml of dioxane were charged into a four-necked glass flask having a net capacity of 500ml, provided with a stirrer, a dropping funnel and a cooling pipe, and heated to the reflux temperature with vigorous stirring. Then, 141g of perfluoro(2-methyl-3, 7-dioxa-8, 9-dibromo-8-nonanamide) obtained in Example 1 (2) was dropwise added thereto. After the dropwise addition, dioxane was removed therefrom by distillation, and 200ml of toluene was added to the residues, and the resulting mixture was heated to the reflux temperature and filtered while hot. Toluene was removed from the filtrate by distillation, whereby 61g of crude perfluoro (2-methyl-3, 7-dioxa-8-nonenamide) was obtained.
(2) 61g of the thus obtained crude perfluoro(2-methyl-37-dioxa-8-nonenamide) and 21g of phosphorus pentoxide were subjected to reaction under the same conditions as in Example 2 (2), whereby 50g of perfluoro(2-methyl-3,7-dioxa-6-nonenonitrile) was obtained (total yield: 52%).

### Reference Example 4 [Synthesis route (C)]

(1) 150g of methyl perfluoro(2-methyl-3,8-dioxa-9-decenoate) having the following chemical formula:

   CF₂=CFO(CF₂)₄OCF(CF₃)COOCH₃

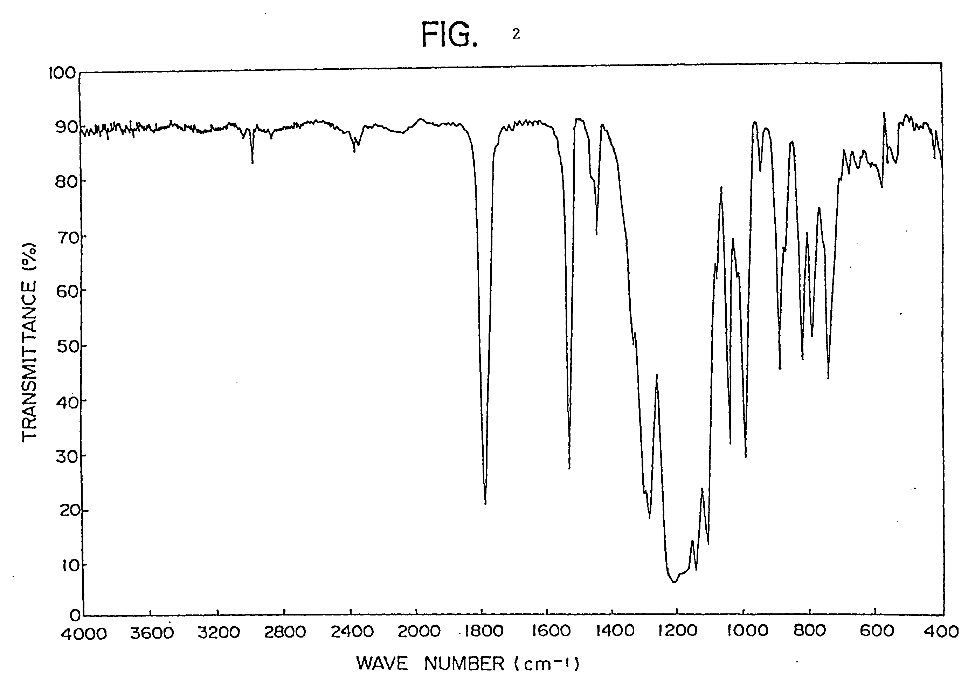

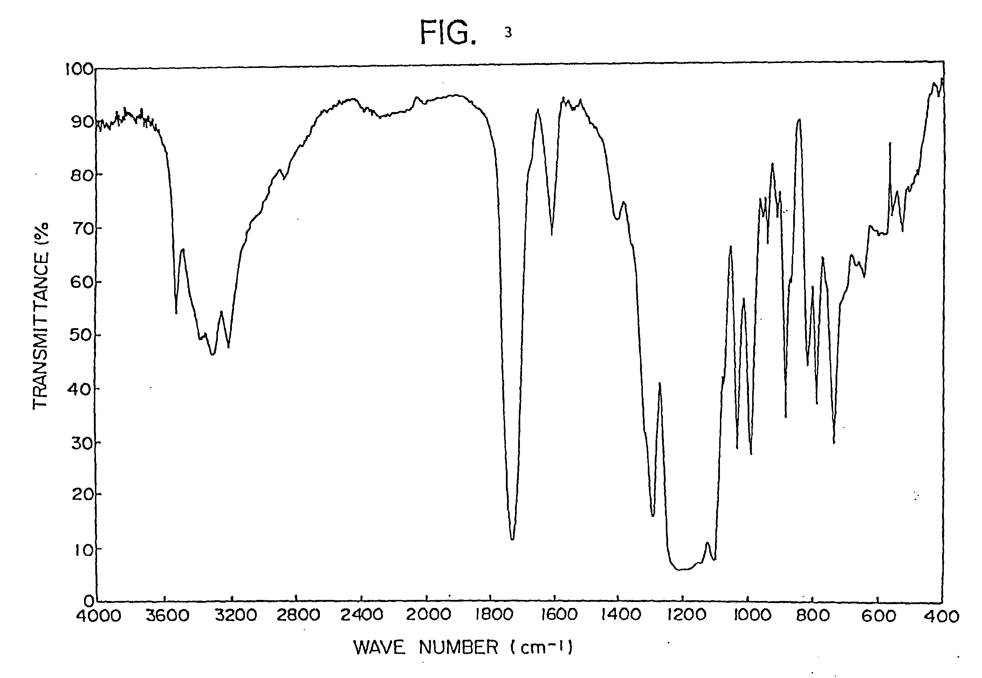

   was charged into a three-necked glass flask having a net capacity of 300ml, provided with a stirrer, a dropping funnel and a cooling pipe, and 67g of bromine was dropwise added thereto from the dropping funnel over one hour, while keeping the flask inside at 5 to 10°C. After the dropwise addition, stirring was further continued at room temperature over a night, and then the reaction mixture was washed with an aqueous 5% sodium hydrogen sulfite solution and then with water. The organic layer was dried over magnesium sulfate, filtered and distilled, whereby 171g of methyl perfluoro(2-methyl-3, 8-dioxa-9, 10-dibromo-9-decanoate) was obtained (yield: 85%).
   Boiling point: 55 ∼ 57°C (0.3 Torr)
   Infrared absorption spectra: see Fig. 2
   ¹⁹F-NMR (internal standard: CFCl₃): 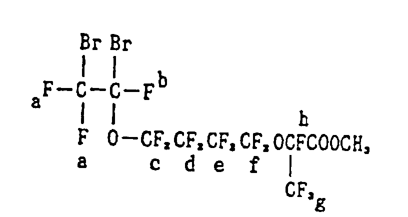
   a =-62.5ppm
   b =-70.2
   c, f =-77.3, -79.1, -84.2, -85.9
   d, e = -124.7
   g = -82.0
   h =-130.6
(2) 23g of the thus obtained methyl perfluoro(2-methyl-3, 8-dioxa-9,10-dibromo-8-nonanoate) and 200ml of perfluorohexane (PF-5060, trademark of a product made by Sumitomo 3M Co., Japan) were charged into a glass flask having a net capacity of 300ml, provided with a stirrer and a gas feed tube, and an ammonia gas was bubbled into the solution at a feed rate of 100ml/min. for one hour with stirring, while keeping the flask inside at 5 to 10°C. Then, the solvent, ammonia and formed methanol were removed therefrom by distillation under reduced pressure, and then the residues were distilled under reduced pressure, whereby 108g of perfluoro(2-methyl-3, 8-dioxa-9, 10-dibromo-9-decanamide) was obtained (yield: 90%).
   Boiling point: 112 ∼ 114°C (1 Torr)
   Infrared absorption spectra: see Fig. 3
(3) 108g of the thus prepared perfluoro(2-methyl-38-dioxa-9,10-dibromo-9-decanamide), 150ml of diglyme and 35.9g of pyridine were charged into a glass flask having a net capacity of 300ml, provided with a stirrer and a dropping funnel, and 47. 8g of trifluoroacetic acid anhydride was dropwise added thereto over one hour, while keeping the flask inside at 5 to 10°C. After the dropwise addition, the temperature of the flask inside was elevated to room temperature and stirring was further continued for 30 minutes. Then, the reaction mixture was poured into 150ml of ice water, and the separated organic layer was washed with water and dried over magnesium sulfate, whereby 93g of perfluoro(2-methyl-38-dioxa-9, 10-dibromo-9-decanonitrile) was obtained (yield: 84%).
(4) 19g of zinc powder, 0.3g of zinc bromide and 150ml of dioxane were charged into a glass flask having a net capacity of 300ml, provided with a stirrer, a dropping funnel and a cooling pipe, and 87g of the thus prepared perfluoro(2-methyl-3, 8-dioxa-9, 10-dibromo-9-decanonitrile) was dropwised added thereto over 30 minutes, while keeping the flask inside at 90 to 100°C. After the dropwise addition, a crude product mixture obtained, and then distilled over phosphorus pentoxide, whereby 54g of perfluoro (2-methyl-3, 8-dioxa-9-decenonitrile) was obtained (yield: 85%).
   Boiling point: 49 ∼ 50°C (72 Torr)
   ¹⁹F-NMR (internal standard: CFCl₃): 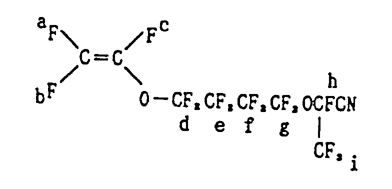
   a =-113.9ppm (dd,Jac=66Hz,Jab=87Hz)
   b =-121.4 (dd,Jab=87Hz,Jbc=112Hz)
   c =-135.1 (dd,Jac=66Hz,Jbc=112Hz)
   d =-83.9 (s)
   e, f =-124.0 (s)
   g =-79.9, -83.9 (AB,J=153Hz)
   h =-113.0 (s)
   i=-88.1 (s)

## Claims

1. A process for producing a perfluoro unsaturated carboxylic acid amide compound represented by the following general formula:
CF₂ = CFO(CF₂)ₙOCF(CF₃)CONH₂
where n is an integer of 2,3,4 or 5, which comprises reacting a perfluoro unsaturated carboxylic acid ester compound represented by the following general formula:
CF₂ = CFO(CF₂)ₙOCF(CF₃)COOR
where R is an alkyl group having 1 to 10 carbon atoms and n has the same meaning as defined above, with ammonia in the presence of a fluorine-containing solvent at a lower temperature than 0°C.

2. The process according to claim 1 wherein the reaction with ammonia is carried out under atmospheric or superatmospheric pressure.

3. The process according to claim 1 wherein the solvent is a solvent selected from the group consisting of 1,1,2-trichloro-1,2,2-trifluoroethane, perfluoro(2-butyltetrahydrofuran) and perfluorohexane.

4. The process according to claim 1 wherein the reaction with ammonia is carried out while keeping an amount of the ammonia equimolar to that of the perfluoro unsaturated carboxylic acid ester.

## Patentansprüche

1. Verfahren zur Herstellung einer ungesättigten Perfluorcarbonsäureamid-Verbindung der folgenden allgemeinen Formel:
**CF**_{**2**}**=CFO(CF**_{**2**}**)**_{**n**}**OCF(CF**_{**3**}**)CONH**_{**2**}
worin n eine ganze Zahl von 2, 3, 4 oder 5 ist, umfassend die Umsetzung einer ungesättigten Perfluorcarbonsäureester-Verbindung der folgenden allgemeinen Formel:
**CF**_{**2**}**=CFO(CF**_{**2**}**)**_{**n**}**OCF(CF**_{**3**}**)COOR**
worin R eine Alkylgruppe mit 1 bis 10 Kohlenstoffatomen ist und n die oben definierte Bedeutung hat, mit Ammoniak in der Gegenwart eines fluorhaltigen Lösungsmittels bei einer niedrigeren Temperatur als 0°C.

2. Verfahren gemäß Anspruch 1, worin die Reaktion mit Ammoniak unter atmosphärischem oder überatmosphärischem Druck durchgeführt wird.

3. Verfahren gemäß Anspruch 1, wobei das Lösungsmittel ein Lösungsmittel ist, das ausgewählt ist aus der Gruppe, bestehend aus 1,1,2-Trifluor-1,2,2-trifluorethan, Perfluor(2-butyltetrahydrofuran) und Perfluorhexan.

4. Verfahren gemäß Anspruch 1, worin die Reaktion mit Ammoniak durchgeführt wird, während die Menge des Ammoniak äquimolar zu der des ungesättigten Perfluorcarbonsäureesters gehalten wird.

## Revendications

1. Procédé de préparation d'un amide d'acide carboxylique insaturé perfluoré représenté par la formule générale suivante :
CF₂ = CFO(CF₂)ₙOCF(CF₃)CONH₂
dans laquelle n est un nombre entier de 2, 3, 4 ou 5, qui comprend le fait de faire réagir un composé d'ester d'acide carboxylique insaturé perfluoré représenté par la formule générale suivante :
CF₂ = CFO(CF₂)ₙOCF(CF₃)COOR
dans laquelle R est un groupe alkyle ayant 1 à 10 atomes de carbone et n a la même signification que ci-dessus, avec de l'ammoniaque en présence d'un solvant contenant du fluor à une température plus basse que 0°C.

2. Procédé selon la revendication 1, dans lequel la réaction avec l'ammoniaque est effectuée sous pression atmosphérique ou sous une pression supérieure à la pression atmosphérique.

3. Procédé selon la revendication 1, dans lequel le solvant est un solvant choisi dans le groupe constitué par le 1,1,2-trichloro-1,2,2-trifluoroéthane, le perfluoro(2-butyltétrahydrofuranne) et le perfluorohexane.

4. Procédé selon la revendication 1, dans lequel la réaction avec l'ammoniaque est effectuée tout en maintenant une quantité d'ammoniaque équimolaire à celle de l'ester d'acide carboxylique insaturé perfluoré.
